# FASCICULE DE BREVET EUROPEEN

(11) **EP 4 157 517 B1**
(45) Date de publication et mention de la délivrance du brevet: **18.02.2026**
(21) Numéro de dépôt: 21726388.8
(22) Date de dépôt: 18.05.2021
(51) Int. Cl.: B01J 21/04, B01J 23/755, B01J 35/30, B01J 37/02, C07C 5/05, C07C 5/03, C07C 5/10, C10G 45/36, C10G 45/48

(54) **PROCEDE DE PREPARATION D'UN CATALYSEUR COMPRENANT UNE PHASE ACTIVE DE NICKEL REPARTIE EN CROUTE ET UN ALLIAGE NICKEL CUIVRE**
VERFAHREN ZUR HERSTELLUNG EINES KATALYSATORS MIT EINER IN EINER SCHALE VERTEILTEN AKTIVEN NICKELPHASE UND EINER NICKEL-KUPFER-LEGIERUNG
METHOD FOR PREPARING A CATALYST CONTAINING AN ACTIVE NICKEL PHASE DISTRIBUTED IN A SHELL AND A NICKEL-COPPER ALLOY

(30) Priorité: 29.05.2020 FR 2005682
(43) Date de publication de la demande: 05.04.2023
(73) Titulaire: IFP Energies nouvelles, 92500 Rueil-Malmaison (FR)
(72) Inventeur: BOUALLEG, Malika, 92852 RUEIL-MALMAISON CEDEX (FR)
(74) Mandataire: IFP Energies nouvelles
(86) Numéro de dépôt international: PCT/EP2021/063045
(87) Numéro de publication internationale: WO 2021/239496

(56) Documents cités:
- WO-A1-2021/018601
- WO-A2-2005/094418
- US-A1- 2015 099 622
- JANG MIN-SU ET AL: "Facile preparation of egg-shell-type pellet catalysts using immiscibility between hydrophobic solvent and hydrophilic solution: Enhancement of catalytic activity due to position control of metallic nickel inside alumina pellet", APPLIED CATALYSIS A: GENERAL, ELSEVIER, AMSTERDAM, NL, vol. 530, 16 November 2016 (2016-11-16), pages 211 - 216, XP029858638, ISSN: 0926-860X, DOI: 10.1016/J.APCATA.2016.11.025

## Description

### Domaine technique

La présente invention concerne un procédé de préparation d'un catalyseur métallique supporté à base de nickel destiné particulièrement à l'hydrogénation des hydrocarbures insaturés, et plus particulièrement, d'hydrogénation sélective de composés polyinsaturés ou d'hydrogénation des aromatiques.

### Etat de la technique

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la fraction essence C5+ (essences contenant des composés hydrocarbonés ayant 5 atomes de carbone ou plus), en particulier des composés styréniques ou indéniques. Ces composés polyinsaturés sont très réactifs et conduisent à des réactions parasites dans les unités de polymérisation. Il est donc nécessaire de les éliminer avant de valoriser ces coupes. L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants.

Les catalyseurs d'hydrogénation sélective sont généralement à base de métaux du groupe VIII du tableau périodique, de préférence le palladium ou le nickel. Le métal se présente sous la forme de particules métalliques déposées sur un support. La teneur en métal, la taille des particules de métal et la répartition de la phase active dans le support font partie des critères qui ont une importance sur l'activité et la sélectivité des catalyseurs.

La répartition macroscopique des particules métalliques dans le support constitue un critère important, principalement dans le cadre de réactions rapides et consécutives telles que les hydrogénations sélectives. Il est généralement souhaitable que ces éléments se situent dans une croûte à la périphérie du support afin d'éviter les problèmes de transfert de matière intragranulaire pouvant conduire à des défauts d'activité et une perte de sélectivité. De tels catalyseurs sont aussi appelé catalyseurs "eggshell" selon la terminologie anglo-saxonne.

De tels catalyseurs sont largement connus dans le cas des catalyseurs d'hydrogénation sélective à base de palladium. En effet, grâce à la faible teneur en palladium (généralement inférieure à 1 % en poids (1 % pds) de palladium par rapport au catalyseur) et des procédés de préparation adaptés, une croûte fine de palladium à la périphérie des grains de support peut être obtenue (FR2922784, US2010/217052).

Il est souvent proposé de substituer le palladium par le nickel, métal moins actif que le palladium qu'il est donc nécessaire de disposer en plus grande quantité dans le catalyseur. Ainsi, les catalyseurs à base de nickel ont généralement une teneur en métal entre 5 et 50 % pds de nickel par rapport au catalyseur. Dans ces catalyseurs, le nickel est généralement réparti de façon homogène au sein du support. Une des voies d'amélioration possible de ces catalyseurs en termes d'activité et de sélectivité est de contrôler la répartition du nickel au sein du support en déposant le nickel de façon plus concentrée sur une croûte, à la périphérie du support. De tels catalyseurs sont connus de l'état de l'art.

Le document US 4 519 951 décrit un catalyseur de type « eggshell » avec du nickel sur un support poreux ayant un volume poreux des pores dont la taille est inférieure à 11,7 nm d'au moins 0,2 ml/g et un volume poreux des pores dont la taille est supérieure à 11,7 nm d'au moins 0,1 ml/g. Plus de 50 % poids du nickel se trouve dans une croûte dont l'épaisseur est égale à 0,15 fois le rayon du support. Ce catalyseur est utilisé pour l'hydrogénation de matières grasses.

Le document CN101890351 décrit un catalyseur supporté de nickel dans lequel plus de 90 % poids du nickel se trouve dans une croûte de 700 µm d'épaisseur. Le catalyseur est préparé en utilisant une solution ammoniacale pour dissoudre le sel de nickel. Ces catalyseurs sont utilisés dans une application d'hydrogénation sélective.

Le document US2012/0065442 décrit un catalyseur supporté de nickel reparti à la fois sur une croûte d'une épaisseur de 3 à 15 % du diamètre et à coeur, le ratio de concentration en nickel entre la croûte et le cœur étant compris entre 3,0 : 1 et 1,3 : 1. Le dépôt de la phase active de nickel est réalisé par pulvérisation (« *spray coating »* selon la terminologie anglo-saxonne) d'une solution ammoniacale d'un sel de nickel sur le support.

Le document de Jang Min-Su et al. intitulé : « Facile preparation of egg-shell-type pellet catalysts using immiscibility between hydrophobic solvent and hydrophilic solution: Enhancement of catalytic activity due to position control of metallic nickel inside alumina pellet », APPLIED CATALYSIS A: GENERAL, 530(2017), 211-216, divulgue un procédé de préparation d'un catalyseur contenant 5 % en poids de nickel sur un support d'alumine, avec une répartition du nickel concentrée dans un croûte mais aussi présent à cœur du support. Le procédé utilisé comprend une étape de mise en contact du support avec un alcool, tel que le butanol, éventuellement une étape de chauffage à une température au-dessus de la température d'ébullition de l'alcool, une étape d'imprégnation du support avec une solution de nickel, puis une étape de séchage à 150°C et enfin une étape de calcination.

La demande de brevet français déposée sous le n°19/08.719 par la Demanderesse décrit un procédé de préparation d'un catalyseur à base de nickel sur un support d'alumine obtenu selon une méthode bien spécifique, le nickel étant réparti à la fois sur une croûte en périphérie du support, et à cœur du support, l'épaisseur de ladite croûte étant comprise entre 2% et 15% du diamètre du catalyseur. Le procédé de préparation d'un tel catalyseur nécessite d'une part l'utilisation d'un support d'alumine spécifique ayant subi un traitement hydrothermal en présence d'une solution acide, et d'autre part la réalisation d'une étape de traitement hydrothermal après l'ajout d'un additif organique spécifique sur le précurseur de catalyseur.

### Objets de l'invention

La présente invention concerne ainsi un nouveau procédé de préparation d'un catalyseur qui permet l'obtention d'un catalyseur comprenant des performances au moins aussi bonnes, voire meilleures, en terme d'activité et de sélectivité dans le cadre des réactions d'hydrogénation sélective de composés polyinsaturés ou d'hydrogénation des aromatiques, tout en utilisant une quantité de phase de nickel effective inférieure (c'est-à-dire une quantité de nickel se trouvant in-fine en croute en périphérie du support permettant la réalisation des réactions d'hydrogénation sélective ou d'hydrogénation des aromatiques) à celle utilisée typiquement dans l'état de la technique, grâce notamment à une meilleure répartition de la phase active de nickel dans le support, rendant cette dernière plus accessible aux réactifs.

La présente invention a pour objet un procédé de préparation d'un catalyseur comprenant du nickel et du cuivre, à raison de 1 et 50 % en poids en élément nickel par rapport au poids total du catalyseur, et à raison de 0,5 à 15 % en poids en élément cuivre par rapport au poids total du catalyseur, et un support poreux d'alumine, le nickel étant réparti à la fois sur une croûte en périphérie du support, et à cœur du support, l'épaisseur de ladite croûte étant comprise entre 2% et 15% du diamètre du catalyseur, la taille des particules de nickel dans le catalyseur, mesurée sous forme oxyde, étant comprise entre 7 nm et 25 nm , lequel procédé comprend les étapes suivantes :
a) on imprègne le support poreux avec un volume V1 d'une solution de butanol compris entre 0,2 et 0,8 fois le volume poreux total VPT dudit support pour obtenir un support imprégné ;
b) on laisse maturer le support poreux imprégné obtenu à l'issue de l'étape a) pendant 0,5 heure à 40 heures ;
c) on réalise l'enchaînement des sous-étapes suivantes :
   c1) on imprègne soit le support poreux imprégné maturé obtenu à l'issue de l'étape b), soit le précurseur de catalyseur obtenu à l'issue de l'étape d), avec une solution comprenant au moins un précurseur de la phase active de nickel ;
   c2) optionnellement, on sèche le précurseur de catalyseur obtenu à l'issue de l'étape c1) à une température inférieure à 250°C ;
d) on réalise l'enchaînement des sous-étapes suivantes :
   d1) on imprègne soit le support poreux, soit le support poreux imprégné maturé obtenu à l'issue de l'étape b), soit le précurseur de catalyseur obtenu à l'issue de l'étape c), avec au moins une solution contenant au moins un précurseur de cuivre et un précurseur de nickel à une concentration en cuivre voulue pour obtenir sur le catalyseur final une teneur comprise entre 0,5 et 15 % poids en élément cuivre par rapport au poids total du catalyseur final ;
   d2) optionnellement, on sèche le précurseur de catalyseur obtenu à l'issue de l'étape d1) à une température inférieure à 250°C ;
   ladite étape d) étant réalisée, soit avant l'étape a), soit entre les étapes b) et c), soit après l'étape c), étant entendu que :
   i) lorsqu'on réalise l'étape d) avant l'étape a), alors les sous-étapes c2) et d2) sont obligatoires ;
   ii) lorsqu'on réalise l'étape d) entre les étapes b) et c), alors la sous-étape c2) est obligatoire ;
   iii) lorsqu'on réalise l'étape d) après l'étape c), alors la sous-étape d2) est obligatoire.

De manière surprenante, la Demanderesse a découvert que la réalisation d'une étape particulière d'imprégnation d'une solution de butanol sur un support poreux d'alumine, quel que soit son origine, suivie d'une étape de maturation avant l'étape d'ajout du précurseur de la phase active de nickel sur ledit support imprégné maturé, et cela sans réaliser d'étape de séchage intermédiaire entre l'imprégnation du butanol et l'imprégnation du précurseur de la phase active de nickel, permet d'obtenir un catalyseur dans lequel au moins une partie du nickel est répartie sur une croûte à la périphérie du support, l'autre partie du nickel étant répartie au cœur du catalyseur. Sans vouloir être lié par une quelconque théorie, la présence de butanol limite la migration de la phase active de nickel au cœur du support. **En** effet, une partie seulement de la porosité est occupée par le butanol. L'étape de maturation permet à la solution de butanol de migrer à cœur du support et de libérer une « couronne de pores libres » à la périphérie du support accessible par le nickel lors de l'étape d'imprégnation du précurseur de la phase active. De plus, le butanol et l'eau étant peu miscibles, la couche de butanol constitue une barrière limitant la diffusion du nickel à cœur du support. Par ailleurs la présence sur le catalyseur d'un alliage NiCu permet à ces catalyseurs d'être réduits *in situ* dans le réacteur avant hydrogénation et ce à des températures très basses par rapport à la température de réduction classique qui se déroule ainsi ex situ et qui nécessite dès lors une étape en plus de passivation.

De préférence, ledit procédé comprend en outre une étape e) dans laquelle on réduit le précurseur de catalyseur obtenu à l'issue de l'enchaînement des étapes a) à d) par mise en contact dudit précurseur de catalyseur avec un gaz réducteur à une température supérieure ou égale à 150°C et inférieure à 250°C.

De préférence, l'étape b) est réalisée à une température inférieure ou égale à 60°C.

De préférence, à l'étape a), on utilise une solution de n-butanol.

Dans un mode de réalisation selon l'invention, lorsqu'on réalise l'étape d) avant l'étape a), le volume V2 de la solution comprenant au moins un précurseur de la phase active de nickel approvisionné à l'étape c1) est de préférence tel que le V2 = VPT - V1.

Dans un mode de réalisation selon l'invention, dans lequel on réalise l'étape d) entre les étapes b) et c), ou après l'étape c), le volume V2 de la solution comprenant au moins un précurseur de la phase active de nickel approvisionné à l'étape c1) et le volume V3 de la solution comprenant un précurseur de nickel et un précurseur de cuivre approvisionnée à l'étape d1) sont tels que V2 + V3 = VPT - V1.

De préférence, le précurseur de cuivre est choisi parmi l'acétate de cuivre, l'acétylacétonate de cuivre, le nitrate de cuivre, le sulfate de cuivre, le chlorure de cuivre, le bromure de cuivre, l'iodure de cuivre ou le fluorure de cuivre.

De préférence, le précurseur de la phase active de nickel est le nitrate de nickel, le chlorure de nickel, l'acétate de nickel ou le hydroxycarbonate de nickel.

Dans un mode de réalisation selon l'invention, ledit procédé comprend en outre une étape de calcination c2') du précurseur de catalyseur séché obtenu à l'issue de l'étape c2) à une température comprise entre 250°C et 600°C.

De préférence, à l'étape a), ledit volume V1 de ladite solution de butanol est compris entre 0,25 et 0,75 fois le volume poreux total VPT dudit support.

De préférence, les sous-étapes c2) et/ou d2) sont réalisées pendant un temps compris entre 0,5 heure et 12 heures.

### Description de la figure

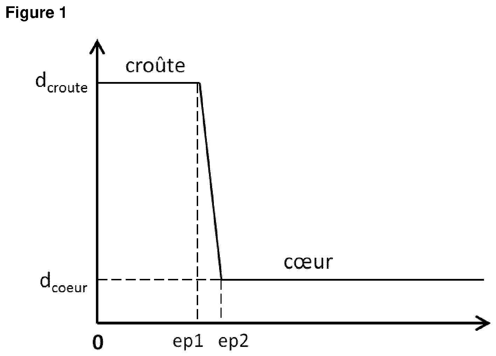

La figure 1 est un schéma représentant la répartition du nickel dans le catalyseur. L'axe des abscisses correspond à l'épaisseur du catalyseur, mesurée depuis le bord du catalyseur (en µm). L'axe des ordonnées correspond à la densité en nickel (en gramme de Ni / mm³). Le nickel est réparti à la fois sur une croûte en périphérie du support, d'épaisseur ep1, et à cœur du support. La densité en nickel sur la croûte d_{croute} est supérieure à la densité en nickel au cœur du support d_{coeur}. L'intervalle de transition entre le cœur et la croûte du catalyseur a une épaisseur notée ep2-ep1.

### Description détaillée de l'invention

### 1. Définitions

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

Dans la présente description, on entend, selon la convention IUPAC, par micropores les pores dont le diamètre est inférieur à 2 nm, c'est à dire 0,002 µm; par mésopores les pores dont le diamètre est supérieur ou égal à 2 nm, c'est à dire 0,002 µm et inférieur ou égal à 50 nm, c'est à dire 0,05 µm et par macropores les pores dont le diamètre est supérieur à 50 nm, c'est à dire 0,05 µm.

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les grains de catalyseur sont enrobés dans des plots de résine époxy. Ces plots sont polis jusqu'à atteindre la coupe au diamètre des billes ou extrudés puis métallisés par dépôt de carbone en évaporateur métallique. La sonde électronique est balayée le long du diamètre de cinq billes ou extrudés pour obtenir le profil de répartition moyen des éléments constitutifs des solides. Cette méthode, bien connue de l'Homme du métier, est définie dans la publication de L. Sorbier et al. "Measurement of palladium crust thickness on catalyst by EPMA" Materials Science and Engineering 32 (2012). Elle permet d'établir le profil de répartition d'un élément donné, ici le nickel, au sein du grain. Par ailleurs, la concentration en Ni est définie pour chaque mesure et donc pour chaque pas d'analyse. La densité de Ni au sein du grain est donc définie comme la concentration de Ni par mm³.

Le volume poreux total est mesuré par porosimétrie au mercure selon la norme ASTM D4284-92 avec un angle de mouillage de 140°, par exemple au moyen d'un appareil modèle Autopore III^{™} de la marque Microméritics^{™}.

La surface spécifique BET est mesurée par physisorption à l'azote selon la norme ASTM D3663-03, méthode décrite dans l'ouvrage Rouquerol F.; Rouquerol J.; Singh K. « Adsorption by Powders & Porous Solids: Principle, methodology and applications », Academic Press, 1999.

On définit également le diamètre médian mésoporeux comme étant le diamètre tel que tous les pores, parmi l'ensemble des pores constituant le volume mésoporeux, de taille inférieure à ce diamètre constituent 50% du volume mésoporeux total déterminé par intrusion au porosimètre à mercure.

On entend par « taille des particules de nickel » le diamètre des cristallites de nickel sous forme oxyde. Le diamètre des cristallites de nickel sous forme oxyde est déterminé par diffraction des rayons X, à partir de la largeur de la raie de diffraction située à l'angle 2thêta=43° (c'est-à-dire selon la direction cristallographique [200]) à l'aide de la relation de Scherrer. Cette méthode, utilisée en diffraction des rayons X sur des poudres ou échantillons polycristallins qui relie la largeur à mi-hauteur des pics de diffraction à la taille des particules, est décrite en détail dans la référence : Appl. Cryst. (1978), 11, 102-113 « Scherrer after sixty years: A survey and some new results in the determination of crystallite size», J. I. Langford and A. J. C. Wilson.

La teneur en nickel et en cuivre est mesurée par fluorescence X.

### 2. Procédé de préparation du catalyseur

Les étapes dudit procédé de préparation sont décrites en détail ci-après.

### Etape a)

Selon l'étape a) du procédé, on imprègne le support d'alumine avec un volume V1 d'une solution de butanol compris entre 0,2 et 0,8 fois le volume poreux total (appelé aussi ici VPT) dudit support à imprégner, de préférence entre 0,25 et 0,75.

Par butanol, on entend les composés organiques comprenant une fonction alcool répondant à la formule chimique brute C₄H₁₀O. On entend ainsi par butanol la famille des composés organiques suivants : le butan-1-ol (ou n-butanol), le butan-2-ol, l'isobutanol, et le tert-butanol. De préférence l'étape a) est réalisée en présence de butan-1-ol.

### Etape b)

Après l'étape a), le support imprégné est maturé à l'état humide pendant 0,5 heure à 40 heures, de manière préférée pendant 1 heure à 30 heures. L'étape b) de maturation est de préférence réalisée à une température inférieure ou égale à 60°C, et plus préférentiellement à température ambiante. Cette étape permet la migration de la solution de butanol au cœur du support.

### Etape c)

### Etape c1)

Lors de l'étape c1) du procédé, on imprègne le support poreux d'alumine imprégné maturé obtenu à l'issue de l'étape b) avec une solution comprenant au moins un précurseur de la phase active de nickel pour obtenir un précurseur de catalyseur. L'étape d'imprégnation peut être réalisée selon des méthodes bien connues de l'Homme du métier.

Le pH de ladite solution comprenant au moins un précurseur de la phase active de nickel imprégné pourra être modifié par l'ajout éventuel d'un acide ou d'une base.

De manière préférée, ledit précurseur de nickel est introduit en solution aqueuse, par exemple sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. De manière préférée, on utilise avantageusement comme précurseur de nickel, le nitrate de nickel, le chlorure de nickel, l'acétate de nickel ou le hydroxycarbonate de nickel. De manière très préférée, le précurseur de nickel est le nitrate de nickel.

La concentration en nickel en solution est ajustée selon le volume poreux du support encore disponible de façon à obtenir pour le catalyseur supporté, une teneur en nickel comprise entre 1 et 50 % poids en élément nickel par rapport au poids total du catalyseur, plus préférentiellement entre 2 et 40 % poids et encore plus préférentiellement entre 3 et 35 % poids et encore plus préférentiellement 5 et 25 % poids.

### Etape c2)

Dans un mode de réalisation selon l'invention, on réalise une étape c2) de séchage du précurseur de catalyseur obtenu à l'issue de l'étape c1). L'étape c2) de séchage est réalisée avantageusement à une température inférieure à 250°C, de préférence comprise entre 15°C et 180°C, plus préférentiellement entre 30°C et 160°C, encore plus préférentiellement entre 50°C et 150°C, et de manière encore plus préférentielle entre 70°C et 140°C, pendant une durée typiquement comprise entre 0,5 heure à 12 heures, et de façon encore plus préférée pendant une durée de 0,5 heure à 5 heures. Des durées plus longues ne sont pas exclues, mais n'apportent pas nécessairement d'amélioration.

L'étape de séchage peut être effectuée par toute technique connue de l'Homme du métier. Elle est avantageusement effectuée sous une atmosphère inerte ou sous une atmosphère contenant de l'oxygène ou sous un mélange de gaz inerte et d'oxygène. Elle est avantageusement effectuée à pression atmosphérique ou à pression réduite. De manière préférée, cette étape est réalisée à pression atmosphérique et en présence d'air ou d'azote.

L'étape c2) n'est pas optionnelle lorsque l'étape d) est réalisée avant l'étape a) ou entre les étapes b) et c). Dans ces cas, à l'issue de l'étape c2), la présence totale, partielle, ou l'absence de la solution de butanol dans le catalyseur n'a pas d'incidence sur l'activité et/ou la sélectivité du catalyseur dans le cadre de l'hydrogénation sélective de composés polyinsaturés ou l'hydrogénation de composés aromatiques.

### Etape c2')

Dans un mode de réalisation selon l'invention, on réalise une étape c2') de calcination du précurseur de catalyseur séché obtenu à l'issue de l'étape c2) à une température comprise entre 250°C et 600°C. L'étape c2') de calcination peut être réalisée à une température comprise entre 250°C et 600°C, de préférence entre 350°C et 550°C, pendant une durée typiquement comprise entre 0,5 heure à 24 heures, de façon préférée pendant une durée de 0,5 heure à 12 heures, et de façon encore plus préférée pendant une durée de 0,5 heure à 10 heures, de préférence sous une atmosphère inerte ou sous une atmosphère contenant de l'oxygène. Des durées plus longues ne sont pas exclues, mais n'apportent pas nécessairement d'amélioration.

A l'issue de l'étape c2'), la présence totale, partielle, ou l'absence de la solution de butanol dans le catalyseur n'a pas d'incidence sur l'activité et/ou la sélectivité du catalyseur dans le cadre de l'hydrogénation sélective de composés polyinsaturés ou l'hydrogénation de composés aromatiques.

### Etape d)

### Etape d1)

Lors de l'étape d1) du procédé, on imprègne soit le support poreux, soit le support poreux imprégné maturé obtenu à l'issue de l'étape b), soit le précurseur de catalyseur obtenu à l'issue de l'étape c), avec au moins une solution contenant au moins un précurseur de cuivre et un précurseur de nickel à une concentration en cuivre voulue pour obtenir sur le catalyseur final une teneur comprise entre 0,5 et 15 % poids en élément cuivre par rapport au poids total du catalyseur final.

Le pH de ladite solution comprenant au moins un précurseur de nickel et un précurseur de cuivre imprégnés pourra être modifié par l'ajout éventuel d'un acide ou d'une base.

De manière préférée, ledit précurseur de nickel et le précurseur de cuivre sont introduits en solution aqueuse.

Lorsque le précurseur de nickel est introduit en solution aqueuse, on utilise avantageusement un précurseur de nickel sous forme de nitrate, de carbonate, d'acétate, de chlorure, d'hydroxyde, d'hydroxycarbonate, d'oxalate, de sulfate, de formiate, de complexes formés par un polyacide ou un acide-alcool et ses sels, de complexes formés avec les acétylacétonates, de complexes tétrammine ou hexammine, ou encore de tout autre dérivé inorganique soluble en solution aqueuse, laquelle est mise en contact avec ledit précurseur de catalyseur. De manière préférée, on utilise avantageusement comme précurseur de nickel, le nitrate de nickel, l'hydroxyde de nickel, le carbonate de nickel, le chlorure de nickel, ou le hydroxycarbonate de nickel. De manière très préférée, le précurseur de nickel est le nitrate de nickel, le carbonate de nickel ou le hydroxyde de nickel.

Lorsque le précurseur de cuivre est introduit en solution aqueuse, on utilise avantageusement un précurseur de cuivre sous forme minérale ou organique. Sous forme minérale, le précurseur de cuivre peut être choisi parmi l'acétate de cuivre, l'acétylacétonate de cuivre, le nitrate de cuivre, le sulfate de cuivre, le chlorure de cuivre, le bromure de cuivre, l'iodure de cuivre ou le fluorure de cuivre. De manière très préférée, le sel précurseur du cuivre est le nitrate de cuivre.

De préférence, le précurseur de nickel est approvisionnée à l'étape d1) à une concentration voulue pour obtenir sur le catalyseur final (i.e. obtenu à l'issue de l'étape e) de réduction) une teneur en nickel comprise dans l'alliage cuivre-nickel comprise entre 0,5 et 15% en poids en élément nickel par rapport au poids total du catalyseur, de préférence entre 1 et 12% en poids, et plus préférentiellement entre 1 et 10% en poids.

Les quantités du ou des précurseurs de cuivre introduites dans la solution selon l'étape d1) sont choisies de telle manière que la teneur totale en cuivre est comprise entre 0,5 et 15 % en poids en élément cuivre par rapport au poids total du catalyseur final (i.e. obtenu à l'issue de l'étape e), de préférence comprise entre 0,5 et 12 % poids, de manière préférée comprise entre 0,75 et 10 % poids, et encore plus préférentiellement entre 1 et 9% en poids.

### Etape d2)

Dans un mode de réalisation selon l'invention, on réalise une étape d2) de séchage du précurseur de catalyseur obtenu à l'issue de l'étape d1). L'étape d2) de séchage est réalisée avantageusement à une température inférieure à 250°C, de préférence comprise entre 15°C et 180°C, plus préférentiellement entre 30°C et 160°C, encore plus préférentiellement entre 50°C et 150°C, et de manière encore plus préférentielle entre 70°C et 140°C, pendant une durée typiquement comprise entre 0,5 heure à 12 heures, et de façon encore plus préférée pendant une durée de 0,5 heure à 5 heures. Des durées plus longues ne sont pas exclues, mais n'apportent pas nécessairement d'amélioration.

L'étape de séchage peut être effectuée par toute technique connue de l'Homme du métier. Elle est avantageusement effectuée sous une atmosphère inerte ou sous une atmosphère contenant de l'oxygène ou sous un mélange de gaz inerte et d'oxygène. Elle est avantageusement effectuée à pression atmosphérique ou à pression réduite. De manière préférée, cette étape est réalisée à pression atmosphérique et en présence d'air ou d'azote.

La sous-étape d2) n'est pas optionnelle lorsque l'étape d) est réalisée avant l'étape a) ou après l'étape c). Lorsque l'étape d) est réalisée après l'étape c), alors à l'issue de la sous-étape d2), la présence totale, partielle, ou l'absence de la solution de butanol dans le catalyseur n'a pas d'incidence sur l'activité et/ou la sélectivité du catalyseur dans le cadre de l'hydrogénation sélective de composés polyinsaturés ou l'hydrogénation de composés aromatiques.

### Etape d2')

Dans un mode de réalisation selon l'invention, on réalise une étape d2') de calcination du précurseur de catalyseur séché obtenu à l'issue de l'étape d2) à une température comprise entre 250°C et 600°C. L'étape d2') de calcination peut être réalisée à une température comprise entre 250°C et 600°C, de préférence entre 350°C et 550°C, pendant une durée typiquement comprise entre 0,5 heure à 24 heures, de façon préférée pendant une durée de 0,5 heure à 12 heures, et de façon encore plus préférée pendant une durée de 0,5 heure à 10 heures, de préférence sous une atmosphère inerte ou sous une atmosphère contenant de l'oxygène. Des durées plus longues ne sont pas exclues, mais n'apportent pas nécessairement d'amélioration.

### Mise en oeuvre des étapes c) et d)

Le procédé de préparation du catalyseur comporte plusieurs modes de mises en œuvre. Ils se distinguent notamment par l'ordre d'introduction de la solution comprenant le précurseur de la phase active de nickel, et de la solution à base de nickel et de cuivre pour l'obtention de l'alliage NiCu.

L'étape d) peut être réalisée soit avant l'étape a), soit entre les étapes b) et c), soit après l'étape c) du procédé de préparation.

Lorsqu'on réalise l'étape d) avant l'étape a), alors les sous-étapes c2) et d2) sont obligatoires. De préférence, on réalise également les étapes c2') et/ou d2'). Dans ce cas, le volume V2 de la solution comprenant au moins un précurseur de la phase active de nickel approvisionné à l'étape c1) est de préférence tel que le V2 = VPT - V1.

Lorsqu'on réalise l'étape d) entre les étapes b) et c), alors la sous-étape c2) est obligatoire. Lorsqu'on réalise l'étape d) après l'étape c), alors la sous-étape d2) est obligatoire. Pour ces deux cas, de préférence, le volume V2 de la solution comprenant au moins un précurseur de la phase active de nickel approvisionné à l'étape c1) et le volume V3 de la solution à base de nickel et de cuivre approvisionnée à l'étape d1) sont tels que V2 + V3 = VPT - V1.

Dans un mode de réalisation selon l'invention, on réalise l'étape d) avant l'étape a).

Dans un autre mode de réalisation selon l'invention, on réalise l'étape d) après l'étape c).

La présence de l'alliage de nickel-cuivre au moins partiellement sous forme réduite permet de recourir à des conditions opératoires de réduction de la phase active de nickel moins sévères que dans l'art antérieur et permet ainsi de réaliser directement l'étape de réduction au sein du réacteur dans lequel on souhaite réaliser l'hydrogénation de composés insaturés ou aromatiques.

Par ailleurs, la présence de cuivre dans le catalyseur permet de conserver une bonne activité du catalyseur et une bonne durée de vie du catalyseur lorsque ce dernier est mis en contact avec une charge hydrocarbonée comprenant du soufre. En effet, par rapport au nickel, le cuivre présent dans le catalyseur capte plus facilement les composés soufrés compris dans la charge, ce qui limite l'empoisonnement irréversible des sites actifs.

### Etape e) Réduction par un gaz réducteur (optionnelle)

Dans un mode de réalisation selon l'invention, préalablement à l'utilisation du catalyseur dans le réacteur catalytique et la mise en œuvre d'un procédé d'hydrogénation, on effectue une étape de traitement réducteur e) en présence d'un gaz réducteur de manière à obtenir un catalyseur comprenant du nickel au moins partiellement sous forme métallique. Cette étape est avantageusement réalisée *in-situ* c'est-à-dire après le chargement du catalyseur dans un réacteur d'hydrogénation. Ce traitement permet d'activer ledit catalyseur et de former des particules métalliques, en particulier du nickel à l'état zéro valent. La réalisation *in-situ* du traitement réducteur du catalyseur permet de s'affranchir d'une étape supplémentaire de passivation du catalyseur par un composé oxygéné ou par le CO₂, ce qui est nécessairement le cas lorsque le catalyseur est préparé en réalisant un traitement réducteur ex-situ, c'est-à-dire en dehors du réacteur utilisé pour l'hydrogénation de composés aromatiques ou polyaromatiques. En effet, lorsque le traitement réducteur est réalisé ex-situ, il est nécessaire de réaliser une étape de passivation afin de préserver la phase métallique du catalyseur en présence d'air (lors des opérations de transport et de chargement du catalyseur dans le réacteur d'hydrogénation), puis de réaliser une étape nouvelle étape de réduction du catalyseur.

Le gaz réducteur est de préférence l'hydrogène. L'hydrogène peut être utilisé pur ou en mélange (par exemple un mélange hydrogène/azote, hydrogène/argon, hydrogène/méthane). Dans le cas où l'hydrogène est utilisé en mélange, toutes les proportions sont envisageables.

Selon un aspect essentiel du procédé de préparation selon l'invention, ledit traitement réducteur est réalisé à une température supérieure ou égale à 150°C et inférieure à 250°C, de préférence comprise entre 160°C et 230°C, et plus préférentiellement entre 170°C et 220°C. La durée du traitement réducteur est comprise entre 5 minutes et moins de 5 heures, de préférence entre 10 minutes et 4 heures, et encore plus préférentiellement entre 10 minutes et 110 minutes.

La montée en température jusqu'à la température de réduction désirée est généralement lente, par exemple fixée entre 0,1 et 10°C/min, de préférence entre 0,3 et 7°C/min.

Le débit d'hydrogène, exprimé en L/heure/gramme de précurseur de catalyseur est compris entre 0,01 et 100 L/heure/gramme de catalyseur, de préférence entre 0,05 et 10 L/heure/gramme de précurseur de catalyseur, de façon encore plus préférée entre 0,1 et 5 L/heure/gramme de précurseur de catalyseur.

### Etape f) Passivation (optionnelle)

Le catalyseur préparé selon le procédé selon l'invention peut avantageusement subir une étape de passivation par un composé soufré qui permet d'améliorer la sélectivité des catalyseurs et d'éviter les emballements thermiques lors des démarrages de catalyseurs neufs *(« run-away »* selon la terminologie anglo-saxonne). La passivation consiste généralement à empoisonner irréversiblement par le composé soufré les sites actifs les plus virulents du nickel qui existent sur le catalyseur neuf et donc à atténuer l'activité du catalyseur en faveur de sa sélectivité. L'étape de passivation est réalisée par la mise en oeuvre de méthodes connues de l'Homme du métier.

L'étape de passivation par un composé soufré est généralement effectuée à une température comprise entre 20°C et 350°C, de préférence entre 40°C et 200°C, pendant 10 minutes à 240 minutes. Le composé soufré est par exemple choisi parmi les composés suivants: thiophène, thiophane, alkylmonosulfures tels que diméthylsulfure, diéthylsulfure, dipropylsulfure et propylméthylsulfure ou encore un disulfure organique de formule HO-R₁-S-S-R₂-OH tel que le di-thio-di-éthanol de formule HO-C₂H₄-S-S-C₂H₄-OH (appelé souvent DEODS). La teneur en soufre est généralement comprise entre 0,1 et 2 % poids dudit élément par rapport au poids total du catalyseur.

Dans un mode de réalisation selon l'invention, la préparation du catalyseur est effectuée ex-situ, c'est-à-dire avant chargement du catalyseur dans l'unité réactionnelle du procédé d'hydrogénation sélective ou d'hydrogénation des aromatiques.

### 3. Catalyseur

Le procédé de préparation selon l'invention permet d'obtenir sur un catalyseur comprenant du nickel et du cuivre, à raison de 1 et 50 % en poids en élément nickel par rapport au poids total du catalyseur, et à raison de 0,5 à 15 % en poids en élément cuivre par rapport au poids total du catalyseur, et un support poreux d'alumine le nickel étant réparti à la fois sur une croûte en périphérie du support, et à cœur du support, l'épaisseur de ladite croûte étant comprise entre 2% et 15% du diamètre du catalyseur, et la taille des particules de nickel dans le catalyseur, mesurée sous forme oxyde, étant comprise entre 7 nm et 25 nm.

De préférence, le nickel est réparti à la fois sur une croûte en périphérie du support, et à cœur du support, l'épaisseur de croûte ep1 étant comprise entre 2,5% et 12% du diamètre du catalyseur, de façon encore plus préférée entre 3% et 10% du diamètre du catalyseur et de façon encore plus préférée entre 3% et 7,5% du diamètre du catalyseur.

De préférence, le ratio de densité en nickel entre la croûte et le cœur (appelé aussi ici d_{croute}/d_{coeur}) est supérieur strictement à 3, de préférence supérieur à 3,5 et de préférence compris entre 3,8 et 15.

De préférence, ladite croûte comprend plus de 25% en poids en élément nickel par rapport au poids total de nickel contenu dans le catalyseur, de préférence plus de 40% en poids, plus préférentiellement entre 45% et 90% en poids, et encore plus préférentiellement entre 60% et 90% en poids.

De préférence, le ratio molaire entre le nickel et le cuivre est compris entre 0,5 mol/mol et 5 mol/mol, de préférence compris entre 0,7 mol/mol et 4,5 mol/mol, plus préférentiellement entre 0,9 mol/mol et 4 mol/mol.

De préférence, au moins une partie du nickel et du cuivre se présente sous la forme d'un alliage de nickel-cuivre, répondant avantageusement à la formule NiₓCu_{y} avec x compris entre 0,1 et 0,9 et y compris entre 0,1 et 0,9.

De préférence, la teneur en nickel comprise dans l'alliage cuivre-nickel est comprise entre 0,5 et 15% en poids en élément nickel par rapport au poids total du catalyseur, de préférence entre 1 et 12% en poids, et plus préférentiellement entre 1 et 10% en poids.

De préférence, la taille des particules de nickel, mesurée sous forme oxyde, dans le catalyseur est comprise entre 7 nm et 25 nm, de préférence entre 8 nm et 23 nm.

Avantageusement, l'intervalle de transition entre le cœur et la croûte du catalyseur (appelé aussi ici intervalle de transition cœur/croûte, ou ep2-ep1 d'après les notations de la figure 1), lié à la variation de la densité de nickel mesurée sur l'épaisseur du catalyseur depuis le bord du catalyseur jusqu'au centre du catalyseur, est très abrupte. De préférence, l'intervalle de transition cœur/ croûte est compris entre 0,05 % et 3 % du diamètre du catalyseur, de préférence entre 0,5 % et 2,5 % du diamètre du catalyseur.

La teneur en nickel dans ledit catalyseur est avantageusement comprise entre 1 et 50 % poids par rapport au poids total du catalyseur, plus préférentiellement entre 2 et 40 % poids et encore plus préférentiellement entre 3 et 35 % poids et encore plus préférentiellement 5 et 25% poids par rapport au poids total du catalyseur.

La teneur en cuivre est comprise entre 0,5 et 15 % en poids en élément cuivre par rapport au poids total du catalyseur, de préférence comprise entre 0,5 et 12 % poids, de manière préférée comprise entre 0,75 et 10 % poids, et encore plus préférentiellement entre 1 et 9 % en poids.

Le catalyseur peut être qualifié comme catalyseur « semi egg-shell » dans lequel la concentration du nickel est plus élevée en périphérie du support que dans le cœur du support, ladite concentration du nickel dans le cœur du support étant non nulle.

La surface spécifique du catalyseur est généralement comprise entre 10 m²/g et 350 m²/g, de préférence entre 25 m²/g et 300 m²/g, de façon plus préférée entre 40 m²/g et 250 m²/g.

Le volume poreux total du catalyseur est généralement compris entre 0,1 ml/g et 1 ml/g, de préférence compris entre 0,2 ml/g et 0,8 ml/g, et de manière particulièrement préférée compris entre 0,3 ml/g et 0,7 ml/g.

De préférence, la phase active du catalyseur ne comprend pas de métal du groupe VIB. Elle ne comprend notamment pas de molybdène ou de tungstène.

Ledit catalyseur (et le support utilisé pour la préparation du catalyseur) est sous forme de grains ayant avantageusement un diamètre compris entre 0,5 mm et 10 mm. Les grains peuvent avoir toutes les formes connues de l'Homme du métier, par exemple la forme de billes (ayant de préférence un diamètre compris entre 1 mm et 8 mm), d'extrudés, de tablettes, de cylindres creux. De préférence, le catalyseur (et le support utilisé pour la préparation du catalyseur) sont sous forme d'extrudés de diamètre compris entre 0,5 mm et 10 mm, de préférence entre 0,8 mm et 3,2 mm et de manière très préférée entre 1,0 mm et 2,5 mm et de longueur comprise entre 0,5 mm et 20 mm. On entend par « diamètre» des extrudés le diamètre du cercle circonscrit à la section droite de ces extrudés. Le catalyseur peut être avantageusement présenté sous la forme d'extrudés cylindriques, multilobés, trilobés ou quadrilobés. De préférence sa forme est trilobée ou quadrilobé. La forme des lobes peut être ajustée selon toutes les méthodes connues de l'art antérieur.

### 4. Support

Les caractéristiques de l'alumine, mentionnées dans cette section, correspondent aux caractéristiques de l'alumine avant la réalisation de l'étape a) du procédé de préparation selon l'invention.

Le support est une alumine c'est-à-dire que le support comporte au moins 95%, de préférence au moins 98%, et de manière particulièrement préférée au moins 99% poids d'alumine par rapport au poids du support. L'alumine présente généralement une structure cristallographique du type alumine delta, gamma ou thêta, seule ou en mélange.

Le support d'alumine, peut comprendre des impuretés telles que les oxydes de métaux des groupes IIA, IIIB, IVB, IIB, IIIA, IVA selon la classification CAS, de préférence la silice, le dioxyde de titane, le dioxyde de zirconium, l'oxyde de zinc, l'oxyde de magnésium et l'oxyde de calcium, ou encore des métaux alcalins, de préférence le lithium, le sodium ou le potassium, et/ou les alcalino-terreux, de préférence le magnésium, le calcium, le strontium ou le baryum ou encore du soufre.

La surface spécifique de l'alumine est généralement comprise entre 10 m²/g et 400m²/g, de préférence entre 30 m²/g et 350 m²/g, de façon plus préférée entre 50 m²/g et 300m²/g.

Le volume poreux total de l'alumine est généralement compris entre 0,1 ml/g et 1,2 ml/g, de préférence compris entre 0,3 ml/g et 0,9 ml/g, et de manière très préférée compris entre 0,5 ml/g et 0,9 ml/g.

### 5. Procédé d'hydrogénation sélective

Il est également décrit un procédé d'hydrogénation sélective de composés polyinsaturés contenant au moins 2 atomes de carbone par molécule, tels que les dioléfines et/ou les acétyléniques et/ou les alcénylaromatiques, aussi appelés styréniques, contenus dans une charge d'hydrocarbures ayant un point d'ébullition final inférieur ou égal à 300°C, lequel procédé étant réalisé à une température comprise entre 0 et 300°C, à une pression comprise entre 0,1 MPa et 10 MPa, à un ratio molaire hydrogène/(composés polyinsaturés à hydrogéner) compris entre 0,1 et 10 et à une vitesse volumique horaire comprise entre 0,1 h⁻¹ et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 h⁻¹ et 40000 h⁻¹ lorsque le procédé est réalisé en phase gazeuse, en présence d'un catalyseur obtenu par le procédé de préparation tel que décrit ci-avant dans la description.

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la coupe C5+ (composés hydrocarbonés ayant au moins 5 atomes de carbone), en particulier des composés dioléfiniques ou styréniques ou indéniques. Ces composés polyinsaturés sont très réactifs et conduisent à des réactions parasites dans les unités de polymérisation. Il est donc nécessaire de les éliminer avant de valoriser ces coupes.

L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants. Dans le cas d'essences de vapocraquage utilisées comme charge, l'hydrogénation sélective permet également d'hydrogéner sélectivement les alcénylaromatiques en aromatiques en évitant l'hydrogénation des noyaux aromatiques.

La charge d'hydrocarbures traitée dans le procédé d'hydrogénation sélective a un point d'ébullition final inférieur ou égal à 300°C et contient au moins 2 atomes de carbone par molécule et comprend au moins un composé polyinsaturé. On entend par « composés polyinsaturés » des composés comportant au moins une fonction acétylénique et/ou au moins une fonction diénique et/ou au moins une fonction alcénylaromatique.

Plus particulièrement, la charge est sélectionnée dans le groupe constitué par une coupe C2 de vapocraquage, une coupe C2-C3 de vapocraquage, une coupe C3 de vapocraquage, une coupe C4 de vapocraquage, une coupe C5 de vapocraquage et une essence de vapocraquage encore appelée essence de pyrolyse ou coupe C5+.

La coupe C2 de vapocraquage, avantageusement utilisée pour la mise en œuvre du procédé d'hydrogénation sélective selon l'invention, présente par exemple la composition suivante : entre 40 et 95 % poids d'éthylène, de l'ordre de 0,1 à 5 % poids d'acétylène, le reste étant essentiellement de l'éthane et du méthane. Dans certaines coupes C2 de vapocraquage, entre 0,1 et 1 % poids de composés en C3 peut aussi être présent.

La coupe C3 de vapocraquage, avantageusement utilisée pour la mise en œuvre du procédé d'hydrogénation sélective selon l'invention, présente par exemple la composition moyenne suivante : de l'ordre de 90 % poids de propylène, de l'ordre de 1 à 8 % poids de propadiène et de méthylacétylène, le reste étant essentiellement du propane. Dans certaines coupes C3, entre 0,1 et 2 % poids de composés en C2 et de composés en C4 peut aussi être présent.

Une coupe C2 - C3 peut aussi être avantageusement utilisée pour la mise en œuvre du procédé d'hydrogénation sélective selon l'invention. Elle présente par exemple la composition suivante : de l'ordre de 0,1 à 5 % poids d'acétylène, de l'ordre de 0,1 à 3 % poids de propadiène et de méthylacétylène, de l'ordre de 30 % poids d'éthylène, de l'ordre de 5 % poids de propylène, le reste étant essentiellement du méthane, de l'éthane et du propane. Cette charge peut aussi contenir entre 0,1 et 2 % poids de composés en C4.

La coupe C4 de vapocraquage, avantageusement utilisée pour la mise en œuvre du procédé d'hydrogénation sélective selon l'invention, présente par exemple la composition massique moyenne suivante : 1 % poids de butane, 46,5 % poids de butène, 51 % poids de butadiène, 1,3 % poids de vinylacétylène et 0,2 % poids de butyne. Dans certaines coupes C4, entre 0,1 et 2 % poids de composés en C3 et de composés en C5 peut aussi être présent.

La coupe C5 de vapocraquage, avantageusement utilisée pour la mise en œuvre du procédé d'hydrogénation sélective selon l'invention, présente par exemple la composition suivante : 21 % poids de pentanes, 45 % poids de pentènes, 34 % poids de pentadiènes.

L'essence de vapocraquage ou essence de pyrolyse, avantageusement utilisée pour la mise en œuvre du procédé d'hydrogénation sélective selon l'invention, correspond à une coupe hydrocarbonée dont la température d'ébullition est généralement comprise entre 0 et 300°C, de préférence entre 10°C et 250°C. Les hydrocarbures polyinsaturés à hydrogéner présents dans ladite essence de vapocraquage sont en particulier des composés dioléfiniques (butadiène, isoprène, cyclopentadiène...), des composés styréniques (styrène, alpha-méthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, une charge formée d'essence de pyrolyse a généralement une composition suivante: 5 à 30 % poids de composés saturés (paraffines et naphtènes), 40 à 80 % poids de composés aromatiques, 5 à 20 % poids de mono-oléfines, 5 à 40 % poids de dioléfines, 1 à 20 % poids de composés alcénylaromatiques, l'ensemble des composés formant 100 %. Elle contient également de 0 à 1000 ppm poids de soufre, de préférence de 0 à 500 ppm poids de soufre.

De manière préférée, la charge d'hydrocarbures polyinsaturés traitée conformément au procédé d'hydrogénation sélective selon l'invention est une coupe C2 de vapocraquage, ou une coupe C2-C3 de vapocraquage, ou une essence de vapocraquage.

Le procédé d'hydrogénation sélective selon l'invention vise à éliminer lesdits hydrocarbures polyinsaturés présents dans ladite charge à hydrogéner sans hydrogéner les hydrocarbures monoinsaturés. Par exemple, lorsque ladite charge est une coupe C2, le procédé d'hydrogénation sélective vise à hydrogéner sélectivement l'acétylène. Lorsque ladite charge est une coupe C3, le procédé d'hydrogénation sélective vise à hydrogéner sélectivement le propadiène et le méthylacétylène. Dans le cas d'une coupe C4, on vise à éliminer le butadiène, le vinylacétylène (VAC) et le butyne, dans le cas d'une coupe C5, on vise à éliminer les pentadiènes. Lorsque ladite charge est une essence de vapocraquage, le procédé d'hydrogénation sélective vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants en évitant l'hydrogénation des noyaux aromatiques.

La mise en œuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs réinjection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en œuvre technologique du procédé d'hydrogénation sélective selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation sélective des coupes C2, C2-C3, C3, C4, C5 et C5+ de vapocraquage peut être réalisée en phase gazeuse ou en phase liquide, de préférence en phase liquide pour les coupes C3, C4, C5 et C5+ et en phase gazeuse pour les coupes C2 et C2-C3. Une réaction en phase liquide permet d'abaisser le coût énergétique et d'augmenter la durée de cycle du catalyseur.

D'une manière générale, l'hydrogénation sélective d'une charge d'hydrocarbures contenant des composés polyinsaturés contenant au moins 2 atomes de carbone par molécule et ayant un point d'ébullition final inférieur ou égal à 300°C s'effectue à une température comprise entre 0 et 300°C, à une pression comprise entre 0,1 MPa et 10 MPa, à un ratio molaire hydrogène/(composés polyinsaturés à hydrogéner) compris entre 0,1 et 10 et à une vitesse volumique horaire (définie comme le rapport du débit volumique de charge sur le volume du catalyseur) comprise entre 0,1 h⁻¹ et 200 h⁻¹ pour un procédé réalisé en phase liquide, ou à un ratio molaire hydrogène/(composés polyinsaturés à hydrogéner) compris entre 0,5 et 1000 et à une vitesse volumique horaire comprise entre 100 h⁻¹ et 40000 h⁻¹ pour un procédé réalisé en phase gazeuse.

Dans un mode de réalisation selon l'invention, lorsqu'on effectue un procédé d'hydrogénation sélective dans lequel la charge est une essence de vapocraquage comportant des composés polyinsaturés, le ratio molaire (hydrogène)/(composés polyinsaturés à hydrogéner) est généralement compris entre 0,5 et 10, de préférence entre 0,7 et 5,0 et de manière encore plus préférée entre 1,0 et 2,0, la température est comprise entre 0 et 200°C, de préférence entre 20°C et 200 °C et de manière encore plus préférée entre 30°C et 180°C, la vitesse volumique horaire (V.V.H.) est comprise généralement entre 0,5 h⁻¹ et 100 h⁻¹, de préférence entre 1 h⁻¹ et 50 h⁻¹ et la pression est généralement comprise entre 0,3 MPa et 8,0 MPa, de préférence entre 1,0 MPa et 7,0 MPa et de manière encore plus préférée entre 1,5 MPa et 4,0 MPa.

Plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 0,7 et 5,0, la température est comprise entre 20°C et 200 °C, la vitesse volumique horaire (V.V.H.) est comprise généralement entre 1 h⁻¹ et 50 h⁻¹ et la pression est comprise entre 1,0 MPa et 7,0 MPa.

Encore plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 1,0 et 2,0, la température est comprise entre 30°C et 180°C, la vitesse volumique horaire (V.V.H.) est comprise généralement entre 1 h⁻¹ et 50 h⁻¹ et la pression est comprise entre 1,5 MPa et 4,0 MPa.

Le débit d'hydrogène est ajusté afin d'en disposer en quantité suffisante pour hydrogéner théoriquement l'ensemble des composés polyinsaturés et de maintenir un excès d'hydrogène en sortie de réacteur.

Dans un autre mode de réalisation selon l'invention, lorsqu'on effectue un procédé d'hydrogénation sélective dans lequel la charge est une coupe C2 de vapocraquage et/ou une coupe C2-C3 de vapocraquage comportant des composés polyinsaturés, le ratio molaire (hydrogène)/(composés polyinsaturés à hydrogéner) est généralement compris entre 0,5 et 1000, de préférence entre 0,7 et 800, la température est comprise entre 0 et 300°C, de préférence entre 15°C et 280 °C, la vitesse volumique horaire (V.V.H.) est comprise généralement entre 100 h⁻¹ et 40000 h⁻¹, de préférence entre 500 h⁻¹ et 30000 h⁻¹ et la pression est généralement comprise entre 0,1 MPa et 6,0 MPa, de préférence entre 0,2 MPa et 5,0 MPa.

### 6. Procédé d'hydrogénation des aromatiques

Il est également décrit un procédé d'hydrogénation d'au moins un composé aromatique ou polyaromatique contenu dans une charge d'hydrocarbures ayant un point d'ébullition final inférieur ou égal à 650°C, généralement compris entre 20°C et 650°C, et de préférence compris entre 20°C et 450°C. Ladite charge d'hydrocarbures contenant au moins un composé aromatique ou polyaromatique peut être choisi parmi les coupes pétrolières ou pétrochimiques suivantes : le reformat du reformage catalytique, le kérosène, le gazole léger, le gazole lourd, les distillats de craquage, tels que l'huile de recyclage de FCC, le gazole d'unité de cokéfaction, les distillats d'hydrocraquage.

La teneur en composés aromatiques ou polyaromatiques contenus dans la charge d'hydrocarbures traitée dans le procédé d'hydrogénation selon l'invention est généralement compris entre 0,1 et 80% en poids, de préférence entre 1 et 50% en poids, et de manière particulièrement préférée entre 2 et 35% en poids, le pourcentage étant basé sur le poids total de la charge d'hydrocarbures. Les composés aromatiques présents dans ladite charge d'hydrocarbures sont par exemple le benzène ou des alkylaromatiques tels que le toluène, l'éthylbenzène, l'o-xylène, le m-xylène, ou le p-xylène, ou encore des aromatiques ayant plusieurs noyaux aromatiques (polyaromatiques) tels que le naphtalène.

La teneur en soufre ou en chlore de la charge est généralement inférieure à 5000 ppm poids de soufre ou de chlore, de préférence inférieure à 100 ppm poids, et de manière particulièrement préférée inférieure à 10 ppm poids.

La mise en oeuvre technologique du procédé d'hydrogénation des composés aromatiques ou polyaromatiques est par exemple réalisée par injection, en courant ascendant ou descendant, de la charge d'hydrocarbures et de l'hydrogène dans au moins un réacteur à lit fixe. Ledit réacteur peut être de type isotherme ou de type adiabatique. Un réacteur adiabatique est préféré. La charge d'hydrocarbures peut avantageusement être diluée par une ou plusieurs ré-injection(s) de l'effluent, issu dudit réacteur où se produit la réaction d'hydrogénation des aromatiques, en divers points du réacteur, situés entre l'entrée et la sortie du réacteur afin de limiter le gradient de température dans le réacteur. La mise en œuvre technologique du procédé d'hydrogénation des aromatiques selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation des composés aromatiques ou polyaromatiques peut être réalisée en phase gazeuse ou en phase liquide, de préférence en phase liquide. D'une manière générale, l'hydrogénation des composés aromatiques ou polyaromatiques s'effectue à une température comprise entre 30°C et 350°C, de préférence entre 50°C et 325°C, à une pression comprise entre 0,1 MPa et 20 MPa, de préférence entre 0,5 MPa et 10 MPa, à un ratio molaire hydrogène/(composés aromatiques à hydrogéner) entre 0,1 et 10 et à une vitesse volumique horaire comprise entre 0,05 h⁻¹ et 50 h⁻¹, de préférence entre 0,1 h⁻¹ et 10 h⁻¹ d'une charge d'hydrocarbures contenant des composés aromatiques ou polyaromatiques et ayant un point d'ébullition final inférieur ou égal à 650°C, généralement compris entre 20°C et 650°C, et de préférence compris entre 20°C et 450°C.

Le débit d'hydrogène est ajusté afin d'en disposer en quantité suffisante pour hydrogéner théoriquement l'ensemble des composés aromatiques et de maintenir un excès d'hydrogène en sortie de réacteur.

La conversion des composés aromatiques ou polyaromatiques est généralement supérieure à 20% en mole, de préférence supérieure à 40% en mole, de manière plus préférée supérieure à 80% en mole, et de manière particulièrement préférée supérieure à 90 % en mole des composés aromatiques ou polyaromatiques contenus dans la charge hydrocarbonée. La conversion se calcule en divisant la différence entre les moles totales des composés aromatiques ou polyaromatiques dans la charge d'hydrocarbures et dans le produit par les moles totales des composés aromatiques ou polyaromatiques dans la charge d'hydrocarbures.

Selon une variante particulière du procédé selon l'invention, on réalise un procédé d'hydrogénation du benzène d'une charge d'hydrocarbures, tel que le reformat issu d'une unité de reformage catalytique. La teneur en benzène dans ladite charge d'hydrocarbures est généralement comprise entre 0,1 et 40% poids, de préférence entre 0,5 et 35% poids, et de manière particulièrement préférée entre 2 et 30% poids, le pourcentage en poids étant basé sur le poids total de la charge d'hydrocarbures.

La teneur en soufre ou en chlore de la charge est généralement inférieure à 10 ppm poids de soufre ou chlore respectivement, et de préférence inférieure à 2 ppm poids.

L'hydrogénation du benzène contenu dans la charge d'hydrocarbures peut être réalisée en phase gazeuse ou en phase liquide, de préférence en phase liquide. Lorsqu'elle est réalisée en phase liquide, un solvant peut être présent, tel que le cyclohexane, l'heptane, l'octane. D'une manière générale, l'hydrogénation du benzène s'effectue à une température comprise entre 30°C et 250°C, de préférence entre 50°C et 200°C, et de manière plus préférée entre 80°C et 180°C, à une pression comprise entre 0,1 MPa et 10 MPa, de préférence entre 0,5 MPa et 4 MPa, à un ratio molaire hydrogène/(benzène) entre 0,1 et 10 et à une vitesse volumique horaire comprise entre 0,05 h⁻¹ et 50 h⁻¹, de préférence entre 0,5 h⁻¹ et 10 h⁻¹.

La conversion du benzène est généralement supérieure à 50% en mole, de préférence supérieure à 80% en mole, de manière plus préférée supérieure à 90% en mole et de manière particulièrement préférée supérieure à 98 % en mole.

L'invention est illustrée via les exemples ci-après qui ne sont nullement limitatifs.

### Exemples

Pour tous les catalyseurs mentionnés dans les exemples ci-après, le support est une alumine A présentant une surface spécifique de 80 m²/g, un volume poreux de 0,7 mL/g et un diamètre médian mésoporeux de 12 nm.

### Exemple 1 : Préparation d'une solution aqueuse de précurseurs de Ni

La solution aqueuse de précurseurs de Ni (solution S1) utilisée pour la préparation des catalyseurs A à H est préparée en dissolvant 43,5 g de nitrate de nickel (NiNO₃, fournisseur Strem Chemicals^{®}) dans un volume de 13 mL d'eau distillée. On obtient la solution S1 dont la concentration en Ni est de 350 g de Ni par litre de solution.

### Exemple 2 : Préparation d'une solution aqueuse des précurseurs de l'alliage NiCu (5%Ni)

La solution aqueuse de précurseurs de l'alliage NiCu (solution S2) utilisée pour la préparation des catalyseurs contenant du NiCu est préparée en dissolvant 14,5 g de nitrate de nickel (NiNO₃, fournisseur Strem Chemicals^{®}) dans un volume de 13 mL d'eau distillée. On obtient une solution dont la concentration en Ni est de 116,6 g de Ni par litre de solution. Le précurseur nitrate de cuivre est ensuite ajouté afin d'avoir un ratio molaire Ni/Cu de 1.

On obtient la solution S2. Elle permet d'introduire les précurseurs de l'alliage NiCu avec une teneur massique en Ni par rapport au catalyseur final de 5% poids par rapport au poids total du catalyseur.

### Exemple 3 : Préparation d'un catalyseur A selon invention [10% poids de Ni +NiCu- ButOH 25% VPT en pré imprégnation]

10 g d'alumine sont imprégnés avec 2,4 ml de butanol ajouté au goutte à goutte. Le support imprégné est ensuite laissé à maturer pendant 30 min à 60°C. Ensuite 7,1 ml de la solution S1 est imprégnée à sec en l'ajoutant goutte-à-goutte, sur le support imprégné maturé obtenu ci-avant. Le précurseur de catalyseur obtenu est ensuite séché en étuve pendant 12 heures à 120°C. Ensuite 10 ml de la solution S2 préparée dans l' exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur A contenant 15 % en poids de l'élément nickel par rapport au poids total du catalyseur (dont 5% en poids en élément nickel compris dans l'alliage NiCu). Les caractéristiques du catalyseur A ainsi obtenu sont reportées dans le tableau 1 ci-après.

### Exemple 4 : Préparation d'un catalyseur B selon invention [5% poids de Ni +NiCu - ButOH 25% VPT en pré-imprégnation]

10 g d'alumine sont imprégnés avec 2,4 ml de butanol ajouté au goutte à goutte. Le support imprégné est ensuite laissé à maturer pendant 30 min à 60°C. 7,1 ml de la solution S1 diluée par deux (3, 35 ml de la solution S1 dilué avec de l'eau pour obtenir 7,1 ml de solution finale, deux fois moins concentrée, que pour l'exemple 3) est imprégnée à sec en l'ajoutant goutte-à-goutte, sur le support imprégné maturé obtenu ci-avant. Le précurseur de catalyseur ainsi obtenu est ensuite séché en étuve pendant 12 heures à 120°C. Ensuite 10 ml de la solution S2 préparée dans l'exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur B contenant 10% en poids de l'élément nickel par rapport au poids total du catalyseur (dont 5% en poids en élément nickel compris dans l'alliage NiCu). Les caractéristiques du catalyseur B ainsi obtenu sont reportées dans le tableau 1 ci-dessous.

### Exemple 5 : Préparation d'un catalyseur C selon invention [10% poids de Ni +NiCu- ButOH 75% VPT d'imprégnation]

10 g d'alumine sont imprégnés avec 7,2 ml de butanol ajouté au goutte à goutte. Le support imprégné est ensuite laissé à maturer pendant 30 min à 60°C. Ensuite 2,4 ml de la solution S1 est imprégnée en l'ajoutant goutte-à-goutte, sur le support imprégné maturé obtenu ci-avant. Le précurseur de catalyseur ainsi obtenu est ensuite séché en étuve pendant 12 heures à 120°C. Ensuite 10 ml de la solution S2 préparée dans l'exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur C contenant 15 % en poids de l'élément nickel par rapport au poids total du catalyseur (dont 5% en poids en élément nickel compris dans l'alliage NiCu). Les caractéristiques du catalyseur C ainsi obtenu sont reportées dans le tableau 1 ci-après.

### Exemple 6 : Préparation d'un catalyseur D non-conforme à l'invention [classique avec NiCu 10% poids Ni]

10 ml de la solution S1 est imprégnée à sec en l'ajoutant goutte-à-goutte, sur le support d'alumine A. Le support imprégné ainsi obtenu est ensuite séché en étuve pendant 12 heures à 120°C. Ensuite 10 ml de la solution S2 préparée dans l'exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur D contenant 15 % en poids de l'élément nickel par rapport au poids total du catalyseur (dont 5% en poids en élément nickel compris dans l'alliage NiCu). Les caractéristiques du catalyseur D ainsi obtenu sont reportées dans le tableau 1 ci-après.

### Exemple 7 : Préparation d'un catalyseur E non-conforme à l'invention [pré-imprégnation ButOH en egg-shell, 10% poids Ni]

10 g d'alumine sont imprégnés avec 2,4 ml de butanol ajouté au goutte à goutte. Le support imprégné est ensuite laissé à maturer pendant 30 min à 60°C. 7,1 ml de la solution S1 préparée à l'exemple 1 est imprégnée à sec, en l'ajoutant goutte-à-goutte, sur le support imprégné maturé obtenu ci-avant.

Le précurseur de catalyseur ainsi obtenu est ensuite séché en étuve pendant 12 heures à 120°C, puis calciné sous un flux d'air sec de 1 L/h/g de catalyseur à 450°C pendant 2 heures.

On obtient le catalyseur E contenant 10% en poids de l'élément nickel par rapport au poids total du catalyseur. Les caractéristiques du catalyseur E ainsi obtenu sont reportées dans le tableau 1 ci-après.

### Exemple 8 : Préparation d'un catalyseur F non-conforme [10% poids de Ni - ButOH 25% VPT en post-imprégnation + NiCul

7,1 ml de la solution S1 est imprégnée à sec en l'ajoutant goutte-à-goutte sur le support d'alumine A. Le précurseur de catalyseur ainsi obtenu est ensuite séché en étuve pendant 12 heures à 120°C. Ensuite 10 ml de la solution S2 préparée à l'exemple 2 est imprégnée à sec en l'ajoutant goutte-à-goutte sur le précurseur de catalyseur obtenu ci-avant.

Le solide ainsi obtenu est ensuite séché en étuve pendant 12 heures à 120°C, puis calciné sous un flux d'air sec de 1 L/h/g de catalyseur à 450°C pendant 2 heures. Sont imprégnés ensuite 2,4 ml de butanol. Le solide est ensuite laissé à maturer pendant 30 min à 60°C. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur F contenant 15 % en poids de l'élément nickel par rapport au poids total du catalyseur (dont 5% en poids en élément nickel compris dans l'alliage NiCu). Les caractéristiques du catalyseur F ainsi obtenu sont reportées dans le tableau 1 ci-après.

### Exemple 9 : Préparation d'un catalyseur G non-conforme [10% poids de Ni - Toluène 25% VPT en pré-imprégnation NiCul

10 g d'alumine imprégnés avec 2,4 ml de toluène ajouté au goutte à goutte. Le support imprégné est ensuite laissé à maturer pendant 30 min à 60°C. Ensuite 7,1 ml de la solution S1 est imprégnée à sec en l'ajoutant goutte-à-goutte, sur le support imprégné maturé obtenu ci-avant. Le précurseur de catalyseur ainsi obtenu est ensuite séché en étuve pendant 12 heures à 120°C. Ensuite 10 ml de la solution S2 préparé dans l'exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur G contenant 15% en poids de l'élément nickel par rapport au poids total du catalyseur (dont 5% en poids en élément nickel compris dans l'alliage NiCu). Les caractéristiques du catalyseur G ainsi obtenu sont reportées dans le tableau 1 ci-après.

### Exemple 10 : Préparation d'un catalyseur H non conforme [10% poids de Ni - n-propanol 25% VPT en pré-imprégnation, NiCul

10 g d'alumine A sont imprégnés avec 2,4 ml de n-propanol ajouté au goutte à goutte. Le support imprégné est ensuite laissé à maturer pendant 30 min à 60°C. Ensuite, 7,1 ml de la solution S1 est imprégnée à sec en l'ajoutant goutte-à-goutte, sur le support imprégné maturé obtenu ci-avant. Le précurseur de catalyseur ainsi obtenu est ensuite séché en étuve pendant 12 heures à 120°C. Ensuite 10 ml de la solution S2 préparé à l'exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur H contenant 15 % en poids de l'élément nickel par rapport au poids total du catalyseur (dont 5% en poids en élément nickel compris dans l'alliage NiCu). Les caractéristiques du catalyseur H ainsi obtenu sont reportées dans le tableau 1 ci-après.

### Exemple 11 : Caractérisation

La quantité d'alliage obtenue après l'étape de calcination puis réduction a été déterminée par analyse par diffraction des rayons X (DRX) sur des échantillons de catalyseur sous forme de poudre.

La quantité de nickel sous forme métallique obtenue après l'étape de réduction a été déterminée par analyse par diffraction des rayons X (DRX) sur des échantillons de catalyseur sous forme de poudre. Entre l'étape de réduction et pendant toute la durée de la caractérisation par DRX les catalyseurs ne sont jamais remis à l'air libre. Les diagrammes de diffraction sont obtenus par analyse radiocristallographique au moyen d'un diffractomètre en utilisant la méthode classique des poudres avec le rayonnement Kα1 du cuivre (λ = 1,5406 Å).

Le taux de réduction a été calculé en calculant l'aire de la raie de Ni⁰ située vers 52°2θ, sur l'ensemble des diffractogrammes de chaque échantillon de catalyseur analysé, puis en soustrayant le signal présent dès la température ambiante sous la raie à 52° et qui est dû à l'alumine.

La tableau 2 ci-après rassemble les taux de réduction ou encore la teneur en nickel métallique Ni° (exprimée en % poids par rapport au poids total de Ni « actif » qui ne compose pas l'alliage) pour tous les catalyseurs A à H caractérisés par DRX après une étape de réduction à 170°C pendant 90 minutes sous flux d'hydrogène. Ces valeurs ont également été comparées avec le taux de réduction obtenu pour le catalyseur E (Ni seul) après une étape de réduction classique (c'est-à-dire à une température de 400°C pendant 15 heures sous flux d'hydrogène).

A température ambiante sur tous les catalyseurs, après calcination, contenant du cuivre et du nickel, nous détectons de l'alumine sous forme delta et thêta, et des grandes raies de NiO et de CuO.

Nous détectons par ailleurs après réduction une raie correspondant à l'alliage sous forme Ni_{0,76}Cu_{0,24.}

Afin d'évaluer le taux de réductibilité et donc la formation du Ni⁰, on mesure l'aire de la raie de Ni⁰ située vers 52°2θ, sur l'ensemble des diffractogrammes, en soustrayant le signal présent dès la température ambiante sous la raie à 52° et qui est dû à l'alumine. On peut ainsi déterminer le pourcentage relatif de Ni⁰ cristallisé après la réduction.

Le tableau 2 ci-dessous récapitule les taux de réductibilité ou encore la teneur en Ni° pour tous les catalyseurs caractérisés par DRX après réduction à 170°C pendant 90 minutes sous flux d'hydrogène. Ces valeurs ont également été comparées avec le taux de réduction obtenu pour le catalyseur E (Ni seul) après une étape de réduction classique (c'est-à-dire à une température de 400°C pendant 15 heures sous flux d'hydrogène).

**Tableau 1**

| Catalyseur | Solvant (% en volume par rapport au VPT du support) | Ni (% pds) | Taille des particules (nm) | Epaisseur de croute / diamètre du grain (%) | d_{croute} / d_{coeur} | Teneur en Ni dans la croûte / Ni total (%) |
|---|---|---|---|---|---|---|
| A (conforme) | 25%BuTOH en pré-imprégnation | 15 | 11,5 | 6,8 | 5 | 66 |
| B (conforme) | 25%BuTOH en pré-imprégnation | 10 | 7 | 3,8 | 12 | 71 |
| C (conforme) | 75%BuTOH en pré-imprégnation | 15 | 12 | 4,7 | 10 | 70 |
| D (non conforme) | - | 15 | 12 | Répartition homogène | - | - |
| E (non conforme) | 25%BuTOH en pré-imprégnation | 10 | 12 | 6,8 | 5 | 66 |
| F (non conforme) | 25%BuTOH en post-imprégnation | 15 | 12 | Répartition homogène | - | - |
| G (non conforme) | 25%toluène en pré-imprégnation | 15 | 11,5 | Répartition homogène | - | - |
| H (non conforme) | 25%n-propanol en pré-imprégnation | 15 | 11,5 | Répartition homogène | - | - |

**Tableau 2**

| Catalyseur | Réduction finale | Ni/Cu | Taille des particules (nm) | Pourcentage de Ni° seul (DRX) après réduction (%) |
|---|---|---|---|---|
| A (conforme) | 170°C, 90 min | oui | 11,5 | 90 |
| B (conforme) | 170°C, 90 min | oui | 7 | 92 |
| C (conforme) | 170°C, 90 min | oui | 12 | 90 |
| D (non conforme) | 170°C, 90 min | oui | 12 | 90 |
| E (non conforme) | 170°C, 90 min | non | 12 | 0* |
| E (non conforme) | 400°C, 15h | non | 12 | 80 |
| F (non conforme) | 170°C, 90 min | oui | 12 | 90 |
| G (non conforme) | 170°C, 90 min | oui | 12 | 95 |
| H (non conforme) | 170°C, 90 min | oui | 12 | 95 |

| | | | | |
|---|---|---|---|---|
| *Nickel sous forme de NiO | | | | |

Pour le catalyseur E (10%Ni seul/alumine), le taux de réductibilité de nickel est de 0% après exactement le même traitement de réduction sous hydrogène que pour les catalyseurs A, B , C, D , F, G et H. Il faut réduire à 400°C le catalyseur E pour avoir une réduction du nickel oxyde en Ni° de l'ordre de 80%.

### Exemple 12 : Tests catalytiques : performances en hydrogénation sélective d'un mélange contenant du styrène et de l'isoprène (A_{HYD1})

Les catalyseurs A à H décrits dans les exemples ci-dessus sont testés vis-à-vis de la réaction d'hydrogénation sélective d'un mélange contenant du styrène et de l'isoprène.

La composition de la charge à hydrogéner sélectivement est la suivante : 8 % pds styrène (fournisseur Sigma Aldrich^{®}, pureté 99%), 8 % pds isoprène (fournisseur Sigma Aldrich^{®}, pureté 99%), 84 % pds n-heptane (solvant) (fournisseur VWR^{®}, pureté > 99% chromanorm HPLC). Cette composition correspond à la composition initiale du mélange réactionnel. Ce mélange de molécules modèles est représentatif d'une essence de pyrolyse.

La réaction d'hydrogénation sélective est opérée dans un autoclave de 500 mL en acier inoxydable, muni d'une agitation mécanique à entraînement magnétique et pouvant fonctionner sous une pression maximale de 100 bar (10 MPa) et des températures comprises entre 5°C et 200°C.

Dans une autoclave sont ajoutés 214 mL de n-heptane (fournisseur VWR^{®}, pureté > 99% chromanorm HPLC) et une quantité de 3 mL de catalyseur. L'autoclave est fermé et purgé. Ensuite l'autoclave est pressurisé sous 35 bar (3,5 MPa) d'hydrogène. Le catalyseur est d'abord réduit *in situ,* à 170°C pendant 90 minutes sous un flux d'hydrogène de 1 L/h/g (rampe de montée en température de 1°C/min) pour les catalyseurs A à H (ce qui correspond ici à l'étape e) du procédé de préparation selon l'invention selon un mode de réalisation). Ensuite l'autoclave est porté à la température du test égale à 30°C. Au temps t=0, environ 30 g d'un mélange contenant du styrène, de l'isoprène, du n-heptane, du pentanethiol et du thiophène sont introduits dans l'autoclave. Le mélange réactionnel a alors la composition décrite ci-dessus et l'agitation est mise en route à 1600 tr/min. La pression est maintenue constante à 35 bar (3,5 MPa) dans l'autoclave à l'aide d'une bouteille réservoir située en amont du réacteur.

Un autre test a été effectué pour le catalyseur E, mais avec une température de réduction du catalyseur de 400°C pendant 16 heures.

L'avancement de la réaction est suivi par prélèvement d'échantillons du milieu réactionnel à intervalles de temps réguliers : le styrène est hydrogéné en éthylbenzène, sans hydrogénation du cycle aromatique, et l'isoprène est hydrogéné en méthyl-butènes. Si la réaction est prolongée plus longtemps que nécessaire, les méthyl-butènes sont à leur tour hydrogénés en isopentane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées (A_{HYD1}) pour les catalyseurs A à H sont reportées dans le tableau 3 ci-après. Elles sont rapportées à l'activité catalytique mesurée pour le catalyseur E préparé dans les conditions classiques de réduction (à une température de 400°C pendant 16 heures sous flux d'hydrogène).

### Exemple 13 : Tests catalytiques : performances en hydrogénation du toluène (A_{HYD2})

Les catalyseurs A à H décrits dans les exemples ci-dessus sont également testés vis-à-vis de la réaction d'hydrogénation du toluène. La réaction d'hydrogénation sélective est opérée dans le même autoclave que celui décrit à l'exemple 12.

Dans une autoclave sont ajoutés 214 mL de n-heptane (fournisseur VWR^{®}, pureté > 99% chromanorm HPLC) et une quantité de 3 mL de catalyseur. L'autoclave est fermé et purgé. Ensuite l'autoclave est pressurisé sous 35 bar (3,5 MPa) d'hydrogène. Le catalyseur est d'abord réduit *in situ,* à 170 °C pendant 90 minutes sous un flux d'hydrogène de 1 L/h/g (rampe de montée en température de 1 °C/min) pour les catalyseurs A à H (ce qui correspond ici à l'étape e) du procédé de préparation selon l'invention selon un mode de réalisation). Après l'ajout de 216 mL de n-heptane (fournisseur VWR^{®}, pureté > 99% chromanorm HPLC), l'autoclave est fermé, purgé, puis pressurisé sous 35 bar (3,5 MPa) d'hydrogène, et porté à la température du test égale à 80°C. Au temps t=0, environ 26 g de toluène (fournisseur SDS^{®}, pureté > 99,8%) sont introduits dans l'autoclave (la composition initiale du mélange réactionnel est alors toluène 6 % pds / n-heptane 94 % pds) et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le toluène est totalement hydrogéné en méthylcyclohexane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées (A_{HYD2}) pour les catalyseurs A à H sont reportées dans le tableau 3 ci-après. Elles sont rapportées à l'activité catalytique mesurée pour le catalyseur E préparé dans les conditions classiques de réduction (à une température de 400°C pendant 16 heures sous flux d'hydrogène).

**Tableau 3**

| Catalyseur | Réduction (°C) | Teneur en Ni° (%) | AHYD1 (%) | A_{HYD2} (%) |
|---|---|---|---|---|
| A (conforme) | 170°C,16h | 15 | 180 | 175 |
| B (conforme) | 170°C,16h | 10 | 150 | 152 |
| C (conforme) | 170°C,16h | 15 | 181 | 190 |
| D (non conforme) | 170°C,16h | 15 | 60 | 70 |
| E (non conforme) | 170°C,16h | 15 | <1 | <1 |
| E (non conforme) | 400°C, 16h | 15 | 100 | 100 |
| F (non conforme) | 170°C,16h | 15 | 55 | 45 |
| G (non conforme) | 170°C,16h | 15 | 93 | 102 |
| H (non conforme) | 170°C,16h | 15 | 95 | 98 |

Ceci montre bien les performances en A_{HYD1} et AH_{YD2} améliorées des catalyseurs A, B et C obtenus par le procédé de préparation selon l'invention, par rapport aux catalyseurs B à H non conformes. Pour les catalyseurs A, B et C, le nickel oxyde est réduit à 170°C à hauteur de 90% et présente des particules réparties en « semi egg-shell ». Le procédé de préparation du catalyseur D ne comprend pas l'ajout de n-butanol en pré-imprégnation ce qui a pour conséquence de ne pas répartir le nickel en semi egg-shell. Le catalyseur E est en retrait d'activité du fait de l'imprégnation classique mis en œuvre sans pré-imprégnation de butanol. Le catalyseur F a subit une post-imprégnation au butanol ce qui ne permet pas une répartition en egg-shell du nickel. Le catalyseur G et H est préparé avec une étape de pré-imprégnation respectivement du toluène et du propanol. Dans ces deux cas, le nickel est réparti de façon homogène dans tout le grain de catalyseur. Les catalyseurs G et H ont dès lors une activité bien en retrait du catalyseur A en A_{HYD1}. Ceci s'explique par la répartition du Ni en « semi egg-shell » sur les catalyseurs A, B, et C qui leur confèrent une activité nettement améliorée notamment dans les réactions rapides d'hydrogénation. Le catalyseur E est en retrait en activité du fait de l'absence de NiCu ce qui ne permet pas d'obtenir du Ni réduit, phase active en hydrogénation, à 170°C.

## Revendications

1. Procédé de préparation d'un catalyseur comprenant du nickel et du cuivre, à raison de 1 et 50 % en poids en élément nickel par rapport au poids total du catalyseur, et à raison de 0,5 à 15 % en poids en élément cuivre par rapport au poids total du catalyseur, et un support poreux d'alumine, le nickel étant réparti à la fois sur une croûte en périphérie du support, et à cœur du support, l'épaisseur de ladite croûte mesuré par microsonde de Castaing étant comprise entre 2% et 15% du diamètre du catalyseur, la taille des particules de nickel dans le catalyseur, mesurée sous forme oxyde et déterminé par diffraction des rayons X, étant comprise entre 7 nm et 25 nm , lequel procédé comprend les étapes suivantes :
a) on imprègne soit le support poreux soit le précurseur de catalyseur obtenu à l'issue de l'étape d) avec un volume V1 d'une solution de butanol compris entre 0,2 et 0,8 fois le volume poreux total VPT dudit support pour obtenir un support imprégné, ledit volume poreux total étant mesuré par porosimétrie au mercure selon la norme ASTM D4284-92 avec un angle de mouillage de 140° ;
b) on laisse maturer le support poreux imprégné obtenu à l'issue de l'étape a) pendant 0,5 heure à 40 heures ;
c) on réalise l'enchaînement des sous-étapes suivantes :
c1) on imprègne soit le support poreux imprégné maturé obtenu à l'issue de l'étape b), soit le précurseur de catalyseur obtenu à l'issue de l'étape d), avec une solution comprenant au moins un précurseur de la phase active de nickel ;
c2) optionnellement, on sèche le précurseur de catalyseur obtenu à l'issue de l'étape c1) à une température inférieure à 250°C ;
d) on réalise l'enchaînement des sous-étapes suivantes :
d1) on imprègne soit le support poreux, soit le support poreux imprégné maturé obtenu à l'issue de l'étape b), soit le précurseur de catalyseur obtenu à l'issue de l'étape c), avec au moins une solution contenant au moins un précurseur de cuivre et un précurseur de nickel à une concentration en cuivre voulue pour obtenir sur le catalyseur final une teneur comprise entre 0,5 et 15 % poids en élément cuivre par rapport au poids total du catalyseur final ;
d2) optionnellement, on sèche le précurseur de catalyseur obtenu à l'issue de l'étape d1) à une température inférieure à 250°C ;
ladite étape d) étant réalisée, soit avant l'étape a), soit entre les étapes b) et c), soit après l'étape c), étant entendu que :
i) lorsqu'on réalise l'étape d) avant l'étape a), alors les sous-étapes c2) et d2) sont obligatoires ;
ii) lorsqu'on réalise l'étape d) entre les étapes b) et c), alors la sous-étape c2) est obligatoire ;
iii) lorsqu'on réalise l'étape d) après l'étape c), alors la sous-étape d2) est obligatoire.

2. Procédé selon la revendication 1, dans lequel l'étape b) est réalisée à une température inférieure ou égale à 60°C.

3. Procédé selon l'une des revendications 1 ou 2, dans lequel à l'étape a) on utilise une solution de n-butanol.

4. Procédé selon l'une quelconque des revendications 1 à 3, dans lequel lorsqu'on réalise l'étape d) avant l'étape a), le volume V2 de la solution comprenant au moins un précurseur de la phase active de nickel approvisionné à l'étape c1) est de préférence tel que le V2 = VPT - V1.

5. Procédé selon l'une quelconque des revendications 1 à 3, dans lequel lorsqu'on réalise l'étape d) entre les étapes b) et c),ou après l'étape c), le volume V2 de la solution comprenant au moins un précurseur de la phase active de nickel approvisionné à l'étape c1) et le volume V3 de la solution comprenant un précurseur de nickel et un précurseur de cuivre approvisionnée à l'étape d1) sont tels que V2 + V3 = VPT - V1.

6. Procédé selon l'une quelconque des revendications 1 à 5, dans lequel le précurseur de cuivre est choisi parmi l'acétate de cuivre, l'acétylacétonate de cuivre, le nitrate de cuivre, le sulfate de cuivre, le chlorure de cuivre, le bromure de cuivre, l'iodure de cuivre ou le fluorure de cuivre.

7. Procédé selon l'une quelconque des revendications 1 à 6, dans lequel le précurseur de la phase active de nickel est le nitrate de nickel, le chlorure de nickel, l'acétate de nickel ou le hydroxycarbonate de nickel.

8. Procédé selon l'une des revendications 1 ou 7, lequel procédé comprenant en outre une étape de calcination c2') du précurseur de catalyseur séché obtenu à l'issue de l'étape c2) à une température comprise entre 250°C et 600°C.

9. Procédé selon l'une quelconque des revendications 1 à 8, dans lequel à l'étape a) ledit volume V1 de ladite solution de butanol est compris entre 0,25 et 0,75 fois le volume poreux total VPT dudit support.

10. Procédé selon l'une quelconque des revendications 1 à 9, dans lequel les sous-étapes c2) et/ou d2) sont réalisées pendant un temps compris entre 0,5 heure et 12 heures.

11. Procédé selon l'une quelconque des revendications 1 à 10, comprenant en outre une étape e) dans laquelle on réduit le précurseur de catalyseur obtenu à l'issue de l'enchaînement des étapes a) à d) par mise en contact dudit précurseur de catalyseur avec un gaz réducteur à une température supérieure ou égale à 150°C et inférieure à 250°C.

## Patentansprüche

1. Verfahren zur Herstellung eines Katalysators, der Nickel und Kupfer in einer Menge von 1 bis 50 Gew.-% des Elements Nickel, bezogen auf das Gesamtgewicht des Katalysators, und in einer Menge von 0,5 bis 15 Gew.-% des Elements Kupfer, bezogen auf das Gesamtgewicht des Katalysators, und einen porösen Aluminiumoxid-Träger umfasst, wobei das Nickel sowohl auf einer Schale am Umfang des Trägers als auch im Kern des Trägers verteilt ist, die mit einer Castaing-Mikrosonde gemessene Dicke der Schale zwischen 2 % und 15 % des Durchmessers des Katalysators beträgt, die Größe der Nickelteilchen im Katalysator, die in der Oxidform gemessen und mittels Röntgenbeugung bestimmt wird, zwischen 7 nm und 25 nm beträgt, wobei das Verfahren die folgenden Schritte umfasst:
a) entweder der poröse Träger oder die aus Schritt d) erhaltene Katalysatorvorstufe wird mit einem Volumen V1 einer Butanollösung mit dem 0,2- bis zum 0,8-Fachen des Gesamtporenvolumens GPV des Trägers getränkt, wodurch ein getränkter Träger erhalten wird, wobei das Gesamtporenvolumen mittels Quecksilberporosimetrie gemäß der Norm ASTM D4284-92 mit einem Benetzungswinkel von 140° gemessen wird;
b) der aus Schritt a) erhaltene getränkte poröse Träger wird 0,5 Stunden bis 40 Stunden lang reifen gelassen;
c) die Abfolge der folgenden Teilschritte wird durchgeführt:
c1) entweder der aus Schritt b) erhaltene gereifte getränkte poröse Träger oder die aus Schritt d) erhaltene Katalysatorvorstufe wird mit einer Lösung getränkt, die mindestens eine Vorstufe der aktiven Nickelphase umfasst;
c2) gegebenenfalls wird die aus Schritt c1) erhaltene Katalysatorvorstufe bei einer Temperatur unter 250 °C getrocknet;
d) die Abfolge der folgenden Teilschritte wird durchgeführt:
d1) entweder der poröse Träger oder der aus Schritt b) erhaltene gereifte getränkte poröse Träger oder die aus Schritt c) erhaltene Katalysatorvorstufe wird mit mindestens einer Lösung getränkt, die mindestens eine Kupfervorstufe und eine Nickelvorstufe mit einer gewünschten Kupferkonzentration zum Erhalt eines Gehalts zwischen 0,5 und 15 Gew.-% des Elements Kupfer, bezogen auf das Gesamtgewicht des endgültigen Katalysators, auf dem endgültigen Katalysator enthält;
d2) gegebenenfalls wird die aus Schritt d1) erhaltene Katalysatorvorstufe bei einer Temperatur unter 250 °C getrocknet;
wobei Schritt d) entweder vor Schritt a) oder zwischen den Schritten b) und c) oder nach Schritt c) durchgeführt wird, mit der Maßgabe, dass:
i) wenn Schritt d) vor Schritt a) durchgeführt wird, die Teilschritte c2) und d2) zwingend erforderlich sind;
ii) wenn Schritt d) zwischen den Schritten b) und c) durchgeführt wird, Teilschritt c2) zwingend erforderlich ist;
iii) wenn Schritt d) nach Schritt c) durchgeführt wird, Teilschritt d2) zwingend erforderlich ist.

2. Verfahren nach Anspruch 1, wobei Schritt b) bei einer Temperatur von weniger als oder gleich 60 °C durchgeführt wird.

3. Verfahren nach einem der Ansprüche 1 oder 2, wobei in Schritt a) eine n-Butanol-Lösung verwendet wird.

4. Verfahren nach einem der Ansprüche 1 bis 3, wobei, wenn Schritt d) vor Schritt a) durchgeführt wird, das Volumen V2 der Lösung, die mindestens eine in Schritt c1) bereitgestellte Vorstufe der aktiven Nickelphase umfasst, vorzugsweise so ist, dass V2 = GPV - V1.

5. Verfahren nach einem der Ansprüche 1 bis 3, wobei, wenn Schritt d) zwischen den Schritten b) und c) oder nach Schritt c) durchgeführt wird, das Volumen V2 der Lösung, die mindestens eine in Schritt c1) bereitgestellte Vorstufe der aktiven Nickelphase umfasst, und das Volumen V3 der Lösung, die eine in Schritt d1) bereitgestellte Nickelvorstufe und Kupfervorstufe umfasst, so sind, dass V2 + V3 = GPV - V1.

6. Verfahren nach einem der Ansprüche 1 bis 5, wobei die Kupfervorstufe aus Kupferacetat, Kupferacetylacetonat, Kupfernitrat, Kupfersulfat, Kupferchlorid, Kupferbromid, Kupferiodid oder Kupferfluorid ausgewählt ist.

7. Verfahren nach einem der Ansprüche 1 bis 6, wobei es sich bei der Vorstufe der aktiven Nickelphase um Nickelnitrat, Nickelchlorid, Nickelacetat oder Nickelhydroxycarbonat handelt.

8. Verfahren nach einem der Ansprüche 1 oder 7, wobei das Verfahren außerdem einen Schritt c2') des Kalzinierens der aus Schritt c2) erhaltenen getrockneten Katalysatorvorstufe bei einer Temperatur zwischen 250 °C und 600 °C umfasst.

9. Verfahren nach einem der Ansprüche 1 bis 8, wobei in Schritt a) das Volumen V1 der Butanollösung zwischen dem 0,25- und dem 0,75-Fachen des Gesamtporenvolumens GPV des Trägers beträgt.

10. Verfahren nach einem der Ansprüche 1 bis 9, wobei die Teilschritte c2) und/oder d2) für einen Zeitraum zwischen 0,5 Stunden und 12 Stunden durchgeführt werden.

11. Verfahren nach einem der Ansprüche 1 bis 10, das außerdem einen Schritt e) umfasst, in dem die am Ende der Abfolge der Schritte a) bis d) erhaltene Katalysatorvorstufe reduziert wird, indem die Katalysatorvorstufe bei einer Temperatur von mehr als oder gleich 150 °C und weniger als 250 °C mit einem reduzierenden Gas in Kontakt gebracht wird.

## Claims

1. Process for preparing a catalyst comprising nickel and copper, in a proportion from 1% to 50% by weight of nickel element relative to the total weight of the catalyst, and in a proportion of 0.5% to 15% by weight of copper element relative to the total weight of the catalyst, and a porous alumina support, the nickel being distributed both over a crust at the periphery of the support, and at the core of the support, the thickness of said crust measured by Castaing microprobe being between 2% and 15% of the diameter of the catalyst, the size of the nickel particles in the catalyst, measured in oxide form and determined by X-ray diffraction, being between 7 nm and 25 nm, which process comprises the following steps:
a) either the porous support or the catalyst precursor obtained at the end of step d) is impregnated with a volume V1 of a butanol solution of between 0.2 and 0.8 times the total pore volume TPV of said support in order to obtain an impregnated support, said total pore volume being measured by mercury porosimetry according to the standard ASTM D4284-92 with a wetting angle of 140°;
b) the impregnated porous support obtained at the end of step a) is left to mature for 0.5 hours to 40 hours;
c) the sequence of the following sub-steps is carried out:
c1) either the matured impregnated porous support obtained at the end of step b), or the catalyst precursor obtained at the end of step d), is impregnated with a solution comprising at least one precursor of the nickel active phase;
c2) optionally, the catalyst precursor obtained at the end of step c1) is dried at a temperature below 250°C;
d) the sequence of the following sub-steps is carried out:
d1) either the porous support, or the matured impregnated porous support obtained at the end of step b), or the catalyst precursor obtained at the end of step c), is impregnated with at least one solution containing at least one copper precursor and one nickel precursor at a desired copper concentration in order to obtain, on the final catalyst, a content of between 0.5% and 15% by weight of copper element relative to the total weight of the final catalyst;
d2) optionally, the catalyst precursor obtained at the end of step d1) is dried at a temperature below 250°C;
said step d) being carried out either before step a), or between steps b) and c), or after step c), it being understood that:
i) when step d) is carried out before step a), then sub-steps c2) and d2) are compulsory;
ii) when step d) is carried out between steps b) and c), then sub-step c2) is compulsory;
iii) when step d) is carried out after step c), then sub-step d2) is compulsory.

2. Process according to Claim 1, wherein said step b) is carried out at a temperature below or equal to 60°C.

3. Process according to either of Claims 1 and 2, wherein, in step a), an n-butanol solution is used.

4. Process according to any one of Claims 1 to 3, wherein, when step d) is carried out before step a), the volume V2 of the solution comprising at least one precursor of the nickel active phase supplied in step c1) is preferably such that V2 = TPV - V1.

5. Process according to any one of Claims 1 to 3, wherein, when step d) is carried out between steps b) and c), or after step c), the volume V2 of the solution comprising at least one precursor of the nickel active phase supplied in step c1) and the volume V3 of the solution comprising a nickel precursor and a copper precursor supplied in step d1) are such that V2 + V3 = TPV - V1.

6. Process according to any one of Claims 1 to 5, wherein the copper precursor is chosen from copper acetate, copper acetylacetonate, copper nitrate, copper sulfate, copper chloride, copper bromide, copper iodide or copper fluoride.

7. Process according to any one of Claims 1 to 6, wherein the precursor of the nickel active phase is nickel nitrate, nickel chloride, nickel acetate or nickel hydroxycarbonate.

8. Process according to one of Claims 1 to 7, which process further comprises a step c2') of calcining the dried catalyst precursor obtained at the end of step c2) at a temperature of between 250°C and 600°C.

9. Process according to any one of Claims 1 to 8, wherein, in step a), said volume V1 of said butanol solution is between 0.25 and 0.75 times the total pore volume TPV of said support.

10. Process according to any one of Claims 1 to 9, wherein sub-steps c2) and/or d2) are carried out for a time of between 0.5 hours and 12 hours.

11. Process according to any one of Claims 1 to 10, further comprising a step e) wherein the catalyst precursor obtained at the end of the sequence of steps a) to d) is reduced by bringing said catalyst precursor into contact with a reducing gas at a temperature above or equal to 150°C and below 250°C.
